# EUROPEAN PATENT SPECIFICATION

(11) **EP 1 293 196 B1**
(45) Date of publication and mention of the grant of the patent: **29.03.2006**
(21) Application number: 02291474.1
(22) Date of filing: 13.06.2002
(51) Int. Cl.: A61K 9/22, A61K 9/32, A61K 31/505, A61K 31/517, A61P 9/12

(54) **Pharmaceutical composition comprising doxazosin**
Pharmazeutische Zubereitung enthaltend Doxazosin
Composition pharmaceutique comprenant de la doxazosine

(30) Priority: 14.09.2001 US 953072
(43) Date of publication of application: 19.03.2003
(73) Proprietor: Cimex Pharma AG, 4102 Benningen/Basel (CH)
(72) Inventor: Seth, Pawan, Irvine, California 92612 (US)
(74) Representative: Pochart, François

(56) References cited:
- WO-A-97/18814
- US-A- 4 765 989

## Description

### BACKGROUND OF THE INVENTION

Compared to conventional pharmaceutical forms, sustained-release forms have various advantages, among which are a decrease in the number of daily doses of medicinal products, hence increased comfort for the patient and better compliance to the treatment, and decreased plasmatic peaks with a reduction in the undesirable effects which preferentially occur during these peaks. This reduction in undesirable effects is particularly desirable with regard to substances with a low therapeutic index (ratio between therapeutic and toxic plasmatic concentrations). The present invention describes the preparation of such a formulation.

Several formulations for sustained release of doxazosin are described in the literature. An osmotic pump formulation is described in US-P-4765989: the active substance is mixed with an osmotically active gelling substance and the entire mixture is coated with a formulation based on a polymer which is impermeable to the active principle but permeable to water. One or more exit holes are made in the coating so as to allow the release of the active principle when the polymer swells. This type of formulation, although advantageous in terms of the kinetics of release which it produces, has the drawback, however, of being highly expensive to produce due to the complex technology implemented (in particular piercing the exit hole for the active principle using a laser beam). US-P-5849240 and US-P-5965163 describe a process for manufacturing mini granules containing doxazosin and a matrix composed of two polymers, one hydrophilic and the other hydrophobic, which is fusible at a temperature ranging, depending on the formulae, between 35 and 150°C. However, and as is often the case for matrixes containing a hydrophobic substance, the examples described in this patent clearly show that, on the one hand, the release profile is far from being linear and that, on the other hand, only a fraction of the active principle (83 to 89% in the examples provided) is released. US-P-6210712 describes an osmotic pump, which combines an osmotically active hydrophilic substance (polyethylene oxide) with the active principle. The whole mixture is coated with a mixture of ethylcellulose (water-impermeable) and hydroxypropylcellulose (permeable). A second coating, permeable to water but not to the active principle, is applied over this entire combination. This formulation also has the drawback of quite complex technology (2 coatings and piercing of the exit hole for the active principle). WO 97/18814 provides a control release pharmaceutical formulations for oral administration consisting essentially of an active compound; low molecular weight polyethylene oxide, HPMC and tabletting excipients formulations which produce a constant rate of release of drug in *in vitro* model of the gastrointestinal tract.

None of the above documents teaches or suggests the present invention.

### SUMMARY OF THE INVENTION

The invention relates to a novel sustained release pharmaceutical composition of doxazosin. Here, doxazosin is intended to cover both the base form and salts form, notably hydrochloride and monomethanesulfonate.

The invention thus a doxazosin composition comprising: (a) a tablet core comprising from 1 to 5% of doxazosin and 50 to 70% by weight of polyethylene oxide forming a hydrophilic matrix, said core being obtained by compressing granules; and optionally (b) a coating.

### DETAILED DESCRIPTION

The present invention consists in a tablet. The tablet may be enteric-coated or not, and may receive a cosmetic coating to enhance patient's comfort.

The tablet (excluding any coating, also referred to as tablet core) comprises from 0.5 to 10% of doxazosin and 20 to 95% by weight of polyethylene oxide (PEO), based on the tablet (excluding any coating) weight. The amount of doxazosin per dosage form may vary between 2 and 40mg.

Preferably, the proportion of doxasozin is from 1 to 5%. Preferably, the proportion of polyethylene oxide is from 50 to 80%.

PEO will act as a hydrophilic matrix.

According to one embodiment, in the composition according to the invention, the polyethylene oxide has a molecular weight, which varies from 50,000 to 8,000,000, and preferably from 100,000 to 3,000,000. The required molecular weight for the PEO can be obtained by mixing PEO of differing molecular weights, which are available commercially. In one embodiment, the hydrophilic matrix comprises between 20 and 90% by weight of polyethylene oxide with a viscosity at 25°C and at 5% of between 8800 and 17600 cPs, and between 5 and 30% by weight of polyethylene oxide with a viscosity at 25°C and at 5% of between 65 and 90 cPs.

The tablet (core thereof) additionally comprises classical excipients, like (microcrystalline) cellulose, lubricants (e.g. stearic acid, glyceryl behenate, etc.), silicon dioxide, desintegrating agents, water-soluble polymers (like polyvinylpyrrolidone), etc.

The uncoated tablet (also referred to as "tablet core") may be obtained by preparing a mixture of the starting compounds and direct compression. Alternatively, the gelling agent and the active ingredient are granulated together, and the resulting granules, optionally with other excipients, are compressed into a tablet. This wet granulation of the various components followed by compressing into tablets is preferred.

According to this second embodiment, granulation may be achieved as follows.

A first process comprises the following steps:
(i) mixing in the dry state and for a sufficient time, doxazosin, polyethylene oxide and optionally, one or several additives;
(ii) adding solvent, followed by mixing for a sufficient period of time;
(iii) granulating the mixture obtained in step (ii);
(iv) drying the granules thus formed for a sufficient period of time;
(v) optionally adding one of more additives, with mixing in the dry state for a sufficient time, optionally with a further sieving;
(vi) compressing the mixture obtained from the preceding steps to obtain the desired compressed tablet; and
(vii) optionally coating said compressed tablet.

A second process comprises the following steps:
(i) mixing, for a sufficient period of time, doxazosin and, optionally, one of several additives, with a solvent;
(ii) adding polyethyleneoxide and mixing for a sufficient period of time;
(iii) granulating the mixture obtained in step (ii);
(iv) drying the granules thus formed for a sufficient period of time;
(v) optionally adding one of more additives, with mixing in the dry state for a sufficient time, optionally with a further sieving;
(vi) compressing the mixture obtained from the preceding steps to obtain the desired compressed tablet; and
(vii) optionally coating said compressed tablet.

In one embodiment, granulation of step (iii) is carried out by passage through a sieve of suitable mesh size.

In one embodiment, the solvent comprises water, alcohol (e.g. isopropanol), or a mixture thereof.

The cosmetic coating may comprise pigments, and traditional excipients such as hydroxyropylmethylcellulose (HPMC) and polyethyleneglycol.

The enteric coating may comprise based on the weight of the coating, from 30 to 80% of a gastroresistant polymer and from 10 to 40% of a hydrophilic silicon dioxide.

The gastroresistant polymer withstands the acidic medium of the stomach and the duodenum, but will dissolve in the intestines, as soon as the pH reaches a predetermined level (e.g. above 5.5 or above 7). This gastroresistant polymer can be selected from the group consisting in (uncured) poly(meth)acrylic acid, cellulose and alkylcellulose-phtalates. Molecular weight can vary within broad limits as will recognize the skilled man. The term "uncured" is used to differentiate over US-P-5580578. Preferably, it is of the type of Eudragit L30D55.

Hydrophilic silicon dioxide is a known hydrophilic anti-tacking agent, the definition of which is known to the skilled man and can be found in the literature. Syloïd® 244FP is one hydrophilic silicon dioxide available from Grace Chemicals.

The enteric coating may further comprise polyethyleneglycol, present in an amount from 5 to 30% by weight, based on the total weight of the functional coating. Stearic acid, dibutyl sebacate, propylene glycol and/or triethyl citrate can used in lieu of or in addition to polyethyleneglycol.

The coating usually represents from 2 to 10% by weight of the tablet core weight.

The composition of the invention is a sustained release; preferably it provides an effective release of doxazosin for a period of at least 8 hours, preferably at least 12 hours.

### PREFERRED EMBODIMENTS

One preferred embodiment is a tablet comprising:
(a) a core comprising doxazosin, polyethylene oxide, polyvinylpyrrolidone, microcrystalline cellulose; said core being obtained by compressing granules;
(b) a coating which is cosmetic or enteric.

### EXAMPLES

The following examples illustrate the invention without limiting it.

### Example 1.

The following core formulation was prepared

| Ingredient | Amount per dosage unit (mg) |
|---|---|
| Doxazosin mesylate | 4.00 |
| Polyethyleneoxide (Polyox®WSR N60K) | 50.00 |
| Microcrystalline cellulose | 31.90 |
| Povidone K30 | 2.50 |
| Isopropanol | 27.00 |
| Purified water | 3.50 |

The povidone is dissolved in the mixture of water and isopropanol. The doxazosin, microcrystalline cellulose and polyethylene oxide are dry-mixed in a high-speed mixer (Stephan UMC5). The liquid is poured onto the powder mixture and this is blended for 5 minutes. The product in granular form obtained is wet-calibrated on an oscillating granulator (Erweka FGS) equipped with a screen which has a mesh size of 2 mm, and then dried in an incubator at 45°C until a constant mass is obtained. The dry product in granular form is calibrated a second time on a screen, which has a mesh size of 0.8 mm.

The following mixture is then prepared

| Ingredient | Amount per dosage unit (mg) |
|---|---|
| Product in granular form | 167.25 |
| Colloidal silica | 0.9 |
| Sodium stearyl fumarate | 1.70 |

The mixture is compressed on a rotary tablet press (Fette P2), equipped with 7 mm punches and at a hardness close to 70 Newtons.

These tablet cores were then coated with an intermediate coating of the following composition:

| Ingredient | Amount per dosage unit (mg) |
|---|---|
| Tablet core | 169.85 |
| Pharmacoat 606® | 5.50 |
| Titanium dioxide | 1.00 |
| Macrogol 600 | 0.85 |
| Talc | 1.00 |
| Purified water | 50.00 |

The Pharmacoat 606 (HPMC) is weighed and dissolved in 75% of the purified water. The polyethylene glycol 6000, iron oxide and titanium dioxide are weighed and dissolved in the remaining purified water and homogenized with an ultra-turrax. This suspension is mixed with the Pharmacoat solution and passed through a screen, which has a mesh size of 350 *µ*m.

The cores are placed in a Glatt GPCG1 fluidized bed fitted with a bottom spray chamber. After preheating at 46°C for 15 minutes, the suspension is sprayed onto the tablets, using the following spraying parameters:

| | |
|---|---|
| Inlet air temperature | 46°C |
| Product temperature | 43-44°C |
| Spraying rate | 10 g/min |
| Atomizing air pressure | 2.1 bar |
| Air volume | 200 m³/h |

Once the spraying is completed, the coated tablets are allowed to dry at 45°C for 20 minutes.

The tablets thus obtained are subjected to a dissolution assay, in compliance with the European and American pharmacopoeia, according to the following procedure:

| | |
|---|---|
| Tool | Rotating basket |
| Speed | 100 rpm |
| Medium | 0.01M phosphate buffer, pH 6.8 |
| Volume | 1000 ml |
| Measurement | Spectrophotometry at 247 nm |

The results are given in % in the following table.

| Time (hour) | 1 | 2 | 4 | 6 | 8 | 12 | 16 |
|---|---|---|---|---|---|---|---|
| Example 1 | 2.9 | 7.8 | 21.5 | 3.9 | 52.7 | 80.9 | 100 |

### Exemple 2.

The following core granules formulation was prepared

| Ingredient | Amount per dosage unit (mg) |
|---|---|
| Doxazosin mesylate | 4.850 |
| Polyethyleneoxide (Polyox® WSR N1105) | 100.00 |
| Polyethyleneoxide (Polyox® WSR N80) | 25.00 |
| Microcrystalline cellulose | 30.00 |
| Povidone K30 | 7.55 |
| Isopropanol | 45.00 |

The same procedure as in example 1 is followed, except that no water is present.

The following mixture is then prepared

| Ingredient | Amount per dosage unit (mg) |
|---|---|
| Product in granular form | 167.40 |
| Colloidal silica | 0.9 |
| Sodium stearyl fumarate | 1.70 |

The mixture is compressed on a rotary tablet press (Fette P2), equipped with 7 mm punches and at a hardness close to 70 Newtons.

These tablet cores were then coated with an intermediate coating of the following composition:

| Ingredient | Amount per dosage unit (mg) |
|---|---|
| Tablet core | 169.85 |
| Pharmacoat 606® | 5.50 |
| Titanium dioxide | 1.00 |
| Macrogol 600 | 0.85 |
| Talc | 1.00 |
| Purified water | 50.00 |

The Pharmacoat 606 (HPMC) is weighed and dissolved in 75% of the purified water. The polyethylene glycol 6000, iron oxide and titanium dioxide are weighed and dissolved in the remaining purified water and homogenized with an ultra-turrax. This suspension is mixed with the Pharmacoat solution and passed through a screen, which has a mesh size of 350 *µ*m.

The cores are placed in a Glatt GPCG1 fluidized bed fitted with a bottom spray chamber. After preheating at 46°C for 15 minutes, the suspension is sprayed onto the tablets, using the following spraying parameters:

| | |
|---|---|
| Inlet air temperature | 46°C |
| Product temperature | 43-44°C |
| Spraying rate | 10 g/min |
| Atomizing air pressure | 2.1 bar |
| Air volume | 200 m³/h |

Once the spraying is completed, the coated tablets are allowed to dry at 45°C for 20 minutes.

The tablets thus obtained are subjected to a dissolution assay, in compliance with the European and American pharmacopoeia, according to the following procedure:

| | |
|---|---|
| Tool | Rotating basket |
| Speed | 100 rpm |
| Medium | 0.01M phosphate buffer, pH 6.8 |
| Volume | 1000 ml |
| Measurement | Spectrophotometry at 247 nm |

The results are given in % in the following table.

| Time (hour) | 1 | 2 | 3 | 5 | 6 | 8 | 12 |
|---|---|---|---|---|---|---|---|
| Example 1 | 9.7 | 21 | 35 | 67 | 81 | 97 | 101 |

### Example 3.

The following core granules formulation was prepared

| Ingredient | Amount per dosage unit (mg) |
|---|---|
| Doxazosin mesylate | 4.850 |
| Polyethyleneoxide (Polyox® WSR N1105) | 110.00 |
| Microcrystalline cellulose | 37.65 |
| Povidone K30 | 7.50 |
| Isopropanol | 45.00 |

The same procedure as in example 1 is followed, except that no water is present.

The following enteric coating formulation is then applied:

| Ingredient | Amount per dosage unit (mg) |
|---|---|
| Eudragit® L30D55 | 13.30 (Solid) |
| Syloid®244FP | 4.00 |
| Polyethyleneglycol 8000 | 2.70 |
| Purified water | 80.00 |

PEG 8000 is dissolved in 45% of amount of purified water. This solution is added to Eudragit suspension and stirred with paddle stirrer for 45 minutes. Syloïd® 244FP is suspended in the remaining part of water and the suspension is homogenized with a high-speed homogenizer Ultra Turrax® T25. The two suspensions are mixed and the mixture is sprayed onto the tablets in a Vector coating pan, using the following parameters:

| | |
|---|---|
| Inlet air temperature | 55-60°C |
| Outlet air temperature | 40-45°C |
| Spraying rate | 5-8 g/min |
| Spraying pressure | 30 psi |
| Pan speed | 16 rpm |

This coating is uncured, since no oven is used once the coating has been applied.

The tablets thus obtained are subjected to a dissolution assay, in the same conditions as in example 1. The results are given in % in the following table.

| Time (hour) | 1 | 2 | 4 | 6 | 8 | 12 | 16 |
|---|---|---|---|---|---|---|---|
| Example 1 | 8.3 | 16.3 | 34 | 51.1 | 70.3 | 6.7 | 100 |

## Claims

1. A doxazosin composition comprising:
(a) a tablet core comprising from 1 to 5% of doxazosin and 50 to 80% by weight of polyethylene oxide forming a hydrophilic matrix, said core being obtained by compressing granules; and optionally
(b) a coating.

2. The composition according to claim 1, in which the hydrophilic matrix comprises between 20 and 90% by weight of polyethylene oxide with a viscosity at 25°C and at 5% of between 8800 and 17600 cPs, and between 5 and 30% by weight of polyethylene oxide with a viscosity at 25°C and at 5% of between 65 and 90 cPs, the molecular weight of the polyethylene oxide being in the range from 50,000 to 8,000,000.

3. The composition according to any one of claims 1 to 2, in which the amount of doxazosin per dosage form varies between 2 and 40mg.

4. The composition according to any one of claims 1 to 3, in which the tablet core comprises doxazosin, polyethylene oxide, polyvinylpyrrolidone, and microcrystalline cellulose.

5. The composition according to any one of claims 1 to 4, in which the coating is a cosmetic coating.

6. The composition according to any one of claims 1 to 4, in which the coating is an enteric coating.

7. The composition according to claim 6, in which the enteric coating comprises, based on the weight of the coating, from 30 to 80% of a gastroresistant polymer, from 10 to 40% of a hydrophilic silicon dioxide and from 5 to 30% of polyethyleneglycol.

8. A process for preparing the composition of any one of claims 1 to 7, comprising the steps of:
(i) wet granulating the various components; and
(ii) compressing the thus-obtained granules into a tablet; and optionally
(iii) coating said tablet.

9. The process of claim 8, comprising the steps of:
(i) mixing in the dry state and for a sufficient time, doxazosin, polyethylene oxide and optionally, one or several additives;
(ii) adding solvent, followed by mixing for a sufficient period of time;
(iii) granulating the mixture obtained in step (ii);
(iv) drying the granules thus formed for a sufficient period of time;
(v) optionally adding one of more additives, with mixing in the dry state for a sufficient time, optionally with a further sieving;
(vi) compressing the mixture obtained from the preceding steps to obtain the desired compressed tablet; and
(vii) optionally coating said compressed tablet.

10. The process of claim 9, in which the granulation of step (iii) is carried out by passage through a sieve of suitable mesh size.

11. The process of any one of claims 8 to 10, in which the solvent comprises water, alcohol or a mixture thereof.

12. The process of claim 8, comprising the steps of:
(i) mixing, for a sufficient period of time, doxazosin and, optionally, one of several additives, with a solvent;
(ii) adding polyethyleneoxide and mixing for a sufficient period of time;
(iii) granulating the mixture obtained in step (ii);
(iv) drying the granules thus formed for a sufficient period of time;
(v) optionally adding one of more additives, with mixing in the dry state for a sufficient time, optionally with a further sieving;
(vi) compressing the mixture obtained from the preceding steps to obtain the desired compressed tablet; and
(vii) optionally coating said compressed tablet.

## Revendications

1. Composition de doxazosine comprenant :
(a) un noyau de comprimé comprenant de 1 % à 5% de doxazosine et de 50% à 80% en poids de poly(oxyde d'éthylène) formant une matrice hydrophile, ledit noyau étant obtenu en comprimant des granulés ; et éventuellement
(b) un enrobage.

2. Composition selon la revendication 1, dans laquelle la matrice hydrophile comprend entre 20% et 90% en poids de poly(oxyde d'éthylène) de viscosité à 25°C et à 5% comprise entre 8800 et 17600 cPs et entre 5% et 30% en poids de poly(oxyde d'éthylène) de viscosité à 25°C et à 5% comprise entre 65 et 90 cPs, la masse moléculaire du poly(oxyde d'éthylène) étant dans la gamme de 50 000 à 8 000 000

3. Composition selon l'une quelconque des revendications 1 à 2, dans laquelle la quantité de doxazosine par forme posologique varie entre 2 et 40 mg.

4. Composition selon l'une quelconque des revendications 1 à 3, dans laquelle le noyau de comprimé comprend de la doxazosine, du poly(oxyde d'éthylène), de la polyvinylpyrrolidone, et de la cellulose microcristalline.

5. Composition selon l'une quelconque des revendications 1 à 4, dans laquelle l'enrobage est un enrobage cosmétique.

6. Composition selon l'une quelconque des revendications 1 à 4, dans laquelle l'enrobage est un enrobage entérique.

7. Composition selon la revendication 6, dans laquelle l'enrobage entérique comprend, par rapport au poids de l'enrobage, de 30% à 80% d'un polymère gastro-résistant, de 10% à 40% de dioxyde de silicium hydrophile et de 5 à 30% de polyéthylèneglycol.

8. Procédé de préparation de la composition selon l'une quelconque des revendications 1 à 7, comprenant les étapes consistant à :
(i) granuler par voie humide les divers composants ; et
(ii) comprimer les granulés ainsi obtenus en un comprimé ; et éventuellement
(iii) enrober ledit comprimé.

9. Procédé selon la revendication 8, comprenant les étapes consistant à :
(i) mélanger à l'état sec et pendant une durée suffisante la doxazosine, le poly(oxyde d'éthylène) et, éventuellement, un ou plusieurs additifs ;
(ii) ajouter un solvant, puis mélanger pendant une durée suffisante ;
(iii) granuler le mélange obtenu à l'étape (ii) ;
(iv) sécher les granulés ainsi formés pendant une durée suffisante ;
(v) ajouter, éventuellement, un ou plusieurs additifs, en mélangeant à l'état sec pendant une durée suffisante, éventuellement en tamisant à nouveau ;
(vi) comprimer le mélange obtenu aux étapes précédentes pour obtenir le comprimé souhaité ; et
(vii) éventuellement enrober ledit comprimé.

10. Procédé selon la revendication 9, dans lequel la granulation de l'étape (iii) est réalisée par passage à travers un tamis présentant une ouverture de maille appropriée.

11. Procédé selon l'une quelconque des revendications 8 à 10, dans lequel le solvant comprend de l'eau, de l'alcool ou d'un mélange de ceux-ci.

12. Procédé selon la revendication 8, comprenant les étapes consistant à :
(i) mélanger, pendant une durée suffisante, la doxazosine et, éventuellement, un ou plusieurs additifs, avec un solvant ;
(ii) ajouter le poly(oxyde d'éthylène) puis mélanger pendant une durée suffisante ;
(iii) granuler le mélange obtenu à l'étape (ii);
(iv) sécher les granulés ainsi formés pendant une durée suffisante ;
(v) ajouter, éventuellement, un ou plusieurs additifs, en mélangeant à l'état sec pendant une durée suffisante, puis éventuellement en tamisant à nouveau ;
(vi) comprimer le mélange obtenu aux étapes précédentes pour obtenir le comprimé souhaité ; et
(vii) éventuellement enrober ledit comprimé.

## Patentansprüche

1. Doxazosinzusammensetzung umfassend:
(a) einen Tablettenkem, umfassend von 1 bis 5 Gew.-% an Doxazosin und 50 bis 80 Gew.-% an Polyethylenoxid, welches eine hydrophile Matrix bildet, wobei der Kern durch Verpressen von Granalien erhalten wird, und gegebenenfalls
(b) eine Beschichtung.

2. Zusammensetzung gemäß Anspruch 1, worin die hydrophile Matrix zwischen 20 und 90 Gew.-% an Polyethylenoxid mit einer Viskosität bei 25°C und bei 5% von zwischen 8.800 und 17.600 cPs und zwischen 5 und 30 Gew.-% an Polyethylenoxid mit einer Viskosität bei 25°C und bei 5% von zwischen 65 und 90 cPs umfaßt, wobei das Molekulargewicht des Polyethylenoxids im Bereich von 50.000 bis 8.000.000 liegt.

3. Zusammensetzung gemäß einem der Ansprüche 1 bis 2, worin die Menge an Doxazosin pro Dosierungsform zwischen 2 und 40 mg variiert.

4. Zusammensetzung gemäß einem der Ansprüche 1 bis 3, worin der Tablettenkern Doxazosin, Polyethylenoxid, Polyvinylpyrrolidon und mikrokristalline Cellulose umfaßt.

5. Zusammensetzung gemäß einem der Ansprüche 1 bis 4, wobei die Beschichtung eine kosmetische Beschichtung ist.

6. Zusammensetzung gemäß einem der Ansprüche 1 bis 4, wobei die Beschichtung eine enterische Beschichtung ist.

7. Zusammensetzung gemäß Anspruch 6, wobei die enterische Beschichtung, bezogen auf das Gewicht der Beschichtung, von 30 bis 80% eines gastroresistenten Polymers, von 10 bis 40% eines hydrophilen Siliciumdioxids und von 5 bis 30% Polyethylenglykol umfaßt.

8. Verfahren zur Herstellung der Zusammensetzung gemäß einem der Ansprüche 1 bis 7, umfassend die Schritte:
(i) das Naßgranulieren der verschiedenen Komponenten und
(ii) das Verpressen der derart erhaltenen Granalien in eine Tablette; und gegebenenfalls
(iii) das Beschichten der Tablette.

9. Verfahren gemäß Anspruch 8, umfassend die Schritte:
(i) das Mischen im Trockenzustand und für eine ausreichende Zeitdauer von Doxazosin, Polyethylenoxid und gegebenenfalls einem oder mehreren Additiven;
(ii) das Zugeben von Lösungsmittel, gefolgt vom Mischen für eine ausreichende Zeitdauer;
(iii) das Granulieren des in Schritt (ii) erhaltenen Gemisches;
(iv) das Trocknen der derart gebildeten Granalien für eine ausreichende Zeitdauer;
(v) gegebenenfalls das Zugeben von einem von mehreren Additiven unter Mischen im Trockenzustand für eine ausreichende Zeitdauer, gegebenenfalls mit einem weiteren Sieben;
(vi) das Verpressen des aus den vorhergehenden Schritten erhaltenen Gemisches unter Erhalten der gewünschten verpressten Tablette und
(vii) gegebenenfalls das Beschichten der verpressten Tablette.

10. Verfahren gemäß Anspruch 9, worin das Granulieren von Schritt (iii) durch Leiten über ein Sieb geeigneter Maschengröße durchgeführt wird.

11. Verfahren gemäß einem der Ansprüche 8 bis 10, worin das Lösungsmittel Wasser, Alkohol oder ein Gemisch davon umfaßt.

12. Verfahren gemäß Anspruch 8, umfassend die Schritte:
(i) das Mischen für eine ausreichende Zeitdauer von Doxazosin und gegebenenfalls einem von mehreren Additiven mit einem Lösungsmittel;
(ii) das Zugeben von Polyethylenoxid und das Mischen für eine ausreichende Zeitdauer;
(iii) das Granulieren des in Schritt (ii) erhaltenen Gemisches;
(iv) das Trocknen der derart gebildeten Granalien für eine ausreichende Zeitdauer;
(v) gegebenenfalls das Zugeben von einem von mehreren Additiven unter Mischen im Trockenzustand für eine ausreichende Zeitdauer, gegebenenfalls mit einem weiteren Sieben;
(vi) das Verpressen des aus den vorhergehenden Schritten erhaltenen Gemisches unter Erhalten der gewünschten verpressten Tablette und
(vii) gegebenenfalls das Beschichten der verpressten Tablette.
